(19) Europäisches Patentamt
European Patent Office
Office européen des brevets

(11) **EP 2 925 437 B1**

(12) **EUROPEAN PATENT SPECIFICATION**

(45) Date of publication and mention
of the grant of the patent:
**06.09.2017  Bulletin 2017/36**

(21) Application number: **13858847.0**

(22) Date of filing: **25.11.2013**

(51) Int Cl.:
*C01B 39/54* (2006.01)          *B01J 29/85* (2006.01)
*C07C 1/20* (2006.01)           *C10G 35/06* (2006.01)

(86) International application number:
**PCT/US2013/071565**

(87) International publication number:
**WO 2014/085281 (05.06.2014 Gazette 2014/23)**

(54) **SILICOMETALLOPHOSPHATE MOLECULAR SIEVES  AND USE**

SILICOMETALLOPHOSPHAT-MOLEKULARSIEBE UND VERWENDUNG

TAMIS MOLÉCULAIRES À SILICOMÉTALLOPHOSPHATES ET D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2012  US 201213689887**
**30.11.2012  US 201213689893**

(43) Date of publication of application:
**07.10.2015  Bulletin 2015/41**

(73) Proprietor: **UOP LLC**
**Des Plaines, Illinois 60017-5017 (US)**

(72) Inventors:
• **LEWIS, Gregory J.**
**Des Plaines, Illinois 60017-5017 (US)**
• **KNIGHT, Lisa M.**
**Des Plaines, Illinois 60017-5017 (US)**
• **JAKUBCZAK, Paulina**
**Des Plaines, Illinois 60017-5017 (US)**
• **STANCZYK, Justin E.**
**Des Plaines, Illinois 60017-5017 (US)**

(74) Representative: **Boult Wade Tennant**
**Verulam Gardens**
**70 Gray's Inn Road**
**London WC1X 8BT (GB)**

(56) References cited:
EP-B1- 0 158 975      EP-B1- 0 161 489
EP-B1- 1 142 833      WO-A1-2005/113439
US-A- 4 310 440       US-A- 4 440 871
US-A- 4 567 029       US-A- 4 973 785
US-A- 5 200 187       US-A1- 2012 157 741
US-B1- 6 388 159      US-B1- 6 613 302
US-B1- 8 569 558

**Description**

FIELD OF THE INVENTION

This invention relates to a new family of charged microporous silicometallosphosphate molecular sieves designated MAPSO-64. They are represented by the empirical formula of:

$$R^+_r M_m^{n+} EP_x Si_y O_z$$

where M is a divalent metal such as magnesium or zinc, R is an organoammonium cation such as diethyldimethylammonium or ethyltrimethylammonium and E is a trivalent element such as aluminum or gallium.

BACKGROUND OF THE INVENTION

Zeolites are crystalline aluminosilicate compositions which are microporous and which are formed from corner sharing $AlO_2^-$ and $SiO_2$ tetrahedra. Numerous zeolites, both naturally occurring and synthetically prepared are used in various industrial processes. Synthetic zeolites are prepared via hydrothermal synthesis employing suitable sources of Si, Al and structure directing agents such as alkali metals, alkaline earth metals, amines, or organoammonium cations. The structure directing agents reside in the pores of the zeolite and are largely responsible for the particular structure that is ultimately formed. These species balance the framework charge associated with aluminum and can also serve as space fillers. Zeolites are characterized by having pore openings of uniform dimensions, having a significant ion exchange capacity, and being capable of reversibly desorbing an adsorbed phase which is dispersed throughout the internal voids of the crystal without significantly displacing any atoms which make up the permanent zeolite crystal structure. Zeolites can be used as catalysts for hydrocarbon conversion reactions, which can take place on the outside surfaces of the zeolite as well as on internal surfaces within the pores of the zeolite.

It is possible to use essentially the same techniques to produce microporous materials of other compositions, e.g., non-zeolitic compositions. In 1982, Wilson et al. first reported aluminophosphate molecular sieves, typically called AlPOs, which are microporous materials that have many of these same properties of zeolites, although they are silica free, composed of $AlO_2^-$ and $PO_2^+$ tetrahedral, see US 4,310,440. Subsequently, charge was introduced to the neutral aluminophosphate frameworks via the substitution of $SiO_2$ tetrahedra for $PO_2^+$ tetrahedra to produce the typically called SAPO molecular sieves, see US 4440871. Another way to introduce framework charge to neutral aluminophosphates is to substitute $[M^{2+}O_2]^{2-}$ tetrahedra for $AlO_2^-$ tetrahedra, which yield the MeAPO molecular sieves, see US 4567029. It is furthermore possible to introduce framework charge on AlPO-based molecular sieves via the simultaneous introduction of $SiO_2$ and $[M^{2+}O_2]^{2-}$ tetrahedra to the framework, giving MeAPSO molecular sieves as shown in US 4973785. EP0161489B1 describes methods of producing three-dimensional microporous framework structures of $CoO_2^{2-}$, $AlO_2^-$, $PO^{2+}$ and $SiO_2$ tetrahedral oxide units having an empirical formula on an anhydrous basis expressed by the formula $mR:(Co_w Al_x P_y Si_z)O_2$ wherein R represents an organoammoniun cation including in particular tetramethylammonium, tetraethylammonium and tetrapropylammonium.

Applicants have synthesized a new family of charged microporous silicometallophosphate framework materials with MeAPSO compositions designated MAPSO-64. The MAPSO-64 materials have the BPH topology, which contains a 12-ring pore system with a perpendicular 8-ring pore system, see for example http://izasc-mirror.la.asu.edu/fini/xsl/IZA-SC/ftc_fw.xsl?-db=Atlas_main&-lay=fw&-max=25&STC=BPH&-find. With respect to AlPO-based materials, the BPH topology has previously been prepared in a MAPO composition of the material known as STA-5, which uses the complicated triquat template 1, 3, 5-Tris (triethylammoniomethyl) benzene, see Patinec et. al. in Chem. Mater., 11, 2456-2462 (1999). By contrast, the microporous MAPSO-64 materials of the present invention additionally includes Si in the framework and can be prepared with the much simpler structure directing agents diethyldimethylammonium (DEDMA⁺) or ethyltrimethylammonium (ETMA⁺).

SUMMARY OF THE INVENTION

As stated, the present invention relates to a new family of microporous silicometallophosphate molecular sieves designated MAPSO-64. One embodiment of the invention is a microporous crystalline material having a three-dimensional framework of containing $EO_2^-$, $PO_2^+$, $[M^{2+}O_2]^{2-}$ and $SiO_2$ tetrahedral units and an empirical composition in the as synthesized and anhydrous basis expressed by an empirical formula of:

$$R^+_r M_m^{2+} EP_x Si_y O_z$$

where M is at least one framework divalent metal cation selected from the group consisting of $Be^{2+}$, $Mg^{2+}$, $Zn^{2+}$, $Co^{2+}$,

$Mn^{2+}$, $Fe^{2+}$, $Ni^{2+}$, "m" is the mole ratio of M to E and varies from 0.01 to 1.5, R is an organoammonium cation selected from the group consisting of ethyltrimethylammonium ($ETMA^+$), diethyldimethylammonium ($DEDMA^+$) and mixtures thereof, "r" is the mole ratio of R to E and has a value of 0.1 to 2.0, E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0, and "z" is the mole ratio of O to E and has a value determined by the equation:

$$z = (2 \bullet m + r + 3 + 5 \bullet x + 4 \bullet y)/2$$

and is characterized in that it has the x-ray diffraction pattern having at least the d-spacings and intensities set forth in Table A:

Table A

| $2\Theta$ | d(Å) | $I/I_0$ |
|---|---|---|
| 6.81 - 6.61 | 12.98 - 13.34 | m - vs |
| 7.75 - 7.55 | 11.40 - 11.70 | m - vs |
| 13.36 - 13.14 | 6.62 - 6.73 | w - m |
| 13.51 - 13.32 | 6.55 - 6.64 | w - m |
| 14.98 - 14.68 | 5.91 - 6.03 | w |
| 15.51 - 15.34 | 5.71 - 5.77 | w - m |
| 18.99 - 18.79 | 4.67 - 4.72 | w - m |
| 20.26 - 20.07 | 4.38 - 4.42 | w - m |
| 21.50 - 21.24 | 4.13 - 4.18 | w - s |
| 24.10 - 23.84 | 3.69 - 3.73 | w - m |
| 24.30 - 24.03 | 3.66 - 3.70 | w - m |
| 24.57 - 24.23 | 3.62 - 3.67 | w - m |
| 26.83 - 26.51 | 3.32 - 3.36 | w - m |
| 27.59 - 27.42 | 3.23 - 3.25 | w - m |
| 27.86 - 27.59 | 3.20 - 3.23 | w |
| 28.68 - 28.49 | 3.11 - 3.13 | w |
| 28.87 - 28.59 | 3.09 - 3.12 | w |
| 30.06 - 29.76 | 2.97 - 3.00 | w - m |
| 30.27 - 29.96 | 2.95 - 2.98 | w - m |
| 31.03 - 30.70 | 2.88 - 2.91 | w - m |
| 33.80 - 33.54 | 2.65 - 2.67 | w - m |
| 34.06 - 33.80 | 2.63 - 2.65 | w |
| 35.60 - 35.31 | 2.52 - 2.54 | w - m |
| 36.65 - 36.34 | 2.45 - 2.47 | w |
| 38.44 - 38.10 | 2.34 - 2.36 | w |

Another embodiment of the invention is a hydrocarbon conversion process using the above-described molecular sieve as a catalyst. The process comprises contacting at least one hydrocarbon with the molecular sieve at conversion conditions to generate at least one converted hydrocarbon.

Yet another embodiment of the invention is a separation process using the crystalline MAPSO-64 material. The process may involve separating mixtures of molecular species or removing contaminants by contacting a fluid with the MAPSO-64 molecular sieve. Separation of molecular species can be based either on the molecular size (kinetic diameter) or on the degree of polarity of the molecular species. Removing contaminants may be by ion exchange with the molecular sieve.

## DETAILED DESCRIPTION OF THE INVENTION

Applicants have prepared a family of microporous silicometallophosphate materials whose structures exhibit the BPH topology. The instant microporous crystalline material (MAPSO-64) has an empirical composition in the as-synthesized form and on an anhydrous basis expressed by the empirical formula:

$$R^+_r M_m^{2+} E P_x Si_y O_z$$

where M is at least one divalent metal cation and is selected from the group consisting of alkaline earth and transition metals. Specific examples of the M cations include but are not limited to beryllium, magnesium, cobalt (II), manganese, zinc, iron(II), nickel and mixtures thereof. R is an organoammonium cation selected from ethyltrimethylammonium (ETMA$^+$), diethyldimethylammonium (DEDMAand mixtures thereof and "r" is the mole ratio of R to E and varies from 0.1 to 2.0. The value of "m" is the mole ratio of M to E and varies from 0.01 to 1.5, "x" is mole ratio of P to E and varies from 0.5 to 2.5. The ratio of silicon to E is represented by "y" which varies from 0.01 to 1.0. E is a trivalent element which is tetrahedrally coordinated, is present in the framework and is selected from the group consisting of aluminum, gallium, iron(III) and boron. Lastly, "z" is the mole ratio of O to E and is given by the equation:

$$z = (2 \bullet m + r + 3 + 5 \bullet x + 4 \bullet y)/2.$$

The microporous crystalline silicometallophosphate material, MAPSO-64, is prepared by a hydrothermal crystallization of a reaction mixture prepared by combining reactive sources of R, E, phosphorous, M and silicon. When E is aluminum, the sources of include but are not limited to aluminum alkoxides, precipitated aluminas, aluminum metal, aluminum hydroxide, aluminum salts and alumina sols. Specific examples of aluminum alkoxides include, but are not limited to aluminum ortho sec-butoxide and aluminum ortho isopropoxide. Sources of other E elements include but are not limited to organoammonium borates, boric acid, precipitated gallium oxyhydroxide, gallium sulfate, ferric sulfate, and ferric chloride. Sources of phosphorus include, but are not limited to, orthophosphoric acid, phosphorus pentoxide, and ammonium dihydrogen phosphate. Sources of silica include but are not limited to tetraethylorthosilicate, colloidal silica, and precipitated silica. Sources of the M metals include the halide salts, nitrate salts, acetate salts, and sulfate salts of the respective alkaline earth and transition metals. R is an organoammonium cation selected from the group consisting of ETMA$^+$, DEDMA$^+$, and mixtures thereof, and the sources include the hydroxide, chloride, bromide, iodide and fluoride compounds. Specific examples include without limitation ethyltrimethylammonium hydroxide, diethyldimethylammonium hydroxide, and diethyldimethylammonium chloride. In a specific embodiment, R is DEDMA$^+$. In another embodiment, R is at ETMA$^+$. In another embodiment, R is a combination of DEDMA$^+$ and ETMA$^+$.

The reaction mixture containing reactive sources of the desired components can be described in terms of molar ratios of the oxides by the formula:

$$aR_2O : bMO : E_2O_3 : cP_2O_5 : dSiO_2 : eH_2O$$

where "a" varies from 0.75 to 16, "b" varies from 0.01 to 3, "c" varies from 0.8 to 8, "d" varies from 0.01 to 4, and "e" varies from 20 to 800. If alkoxides are used, a distillation or evaporative step may be included to remove the alcohol hydrolysis products. The reaction mixture is now reacted at a temperature of 60°C to 200°C, or in another embodiment, from 115°C to 175°C for a period of 1 day to 3 weeks or in another embodiment, 1 day to 7 days in a sealed reaction vessel under autogenous pressure. After crystallization is complete, the solid product is isolated from the heterogeneous mixture by means such as filtration or centrifugation, and then washed with deionized water and dried in air at ambient temperature up to 100°C. MAPSO-64 seeds can optionally be added to the reaction mixture in order to accelerate the formation of the desired microporous composition.

The MAPSO-64 silicometallophosphate-based material, which is obtained from the above-described process, is characterized by the x-ray diffraction pattern, having at least the d-spacings and relative intensities set forth in Table A below.

Table A

| 2Θ | d(Å) | I/I$_0$ |
|---|---|---|
| 6.81 - 6.61 | 12.98 - 13.34 | m - vs |
| 7.75 - 7.55 | 11.40 - 11.70 | m - vs |
| 13.36 - 13.14 | 6.62 - 6.73 | w - m |
| 13.51 - 13.32 | 6.55 - 6.64 | w - m |
| 14.98 - 14.68 | 5.91 - 6.03 | w |
| 15.51 - 15.34 | 5.71 - 5.77 | w - m |
| 18.99 - 18.79 | 4.67 - 4.72 | w - m |
| 20.26 - 20.07 | 4.38 - 4.42 | w - m |
| 21.50 - 21.24 | 4.13 - 4.18 | w - s |
| 24.10 - 23.84 | 3.69 - 3.73 | w - m |
| 24.30 - 24.03 | 3.66 - 3.70 | w - m |
| 24.57 - 24.23 | 3.62 - 3.67 | w - m |
| 26.83 - 26.51 | 3.32 - 3.36 | w - m |
| 27.59 - 27.42 | 3.23 - 3.25 | w - m |
| 27.86 - 27.59 | 3.20 - 3.23 | w |
| 28.68 - 28.49 | 3.11 - 3.13 | w |
| 28.87 - 28.59 | 3.09 - 3.12 | w |
| 30.06 - 29.76 | 2.97 - 3.00 | w - m |
| 30.27 - 29.96 | 2.95 - 2.98 | w - m |
| 31.03 - 30.70 | 2.88 - 2.91 | w - m |
| 33.80 - 33.54 | 2.65 - 2.67 | w - m |
| 34.06 - 33.80 | 2.63 - 2.65 | w |
| 35.60 - 35.31 | 2.52 - 2.54 | w - m |
| 36.65 - 36.34 | 2.45 - 2.47 | w |
| 38.44 - 38.10 | 2.34 - 2.36 | w |

The MAPSO-64 may be modified in many ways to tailor it for use in a particular application. Modifications include calcination, ammonia calcinations, ion-exchange, steaming, various acid extractions, ammonium hexafluorosilicate treatment, or any combination thereof, as outlined for the case of UZM-4 in US 6,776,975 B1 which is incorporated by reference in its entirety. Properties that may be modified include porosity, adsorption, framework composition, acidity, thermal stability, etc.

As synthesized, the MAPSO-64 material will contain some of the exchangeable or charge balancing cations in its pores. These exchangeable cations can be exchanged for other cations, or in the case of organic cations, they can be removed by heating under controlled conditions. With the large 12-ring pore BPH topology of MAPSO-64, small organoammonium cations can often be removed directly by ion-exchange. In one embodiment, the method of removing organic cations from the pores is ammonia calcination. Calcination in air converts organic cations in the pores to protons, which can, for example, lead to some removal of A1 from the framework upon exposure to water vapor. When the calcination is carried out in an ammonia atmosphere, the organic cation in the pore is replaced by $NH_4^+$ cation and the framework remains intact, see Studies in Surface Science, (2004) vol. 154, p.1324 - 1331. Typical conditions for ammonia calcinations include the use of gaseous anhydrous ammonia flowing at a rate of 1.11/min while ramping the sample at 2 - 5°C/min to 500°C and holding at that temperature for a time ranging from 5 minutes to 4 hours. The resulting ammonium form of MAPSO-64 has essentially the diffraction pattern of Table A. The ammonium form of MAPSO-64 may then be ion-exchanged to any other form, resulting in a material with a modified composition, MAPSO-64M, given by the empirical formula:

$$M'^{p+}_n M^{2+}_m E P_x Si_y O_z$$

where M is at least one divalent metal cation selected from the group consisting of $Be^{2+}$, $Mg^{2+}$, $Zn^{2+}$, $Co^{2+}$, $Mn^{2+}$, $Fe^{2+}$, $Ni^{2+}$, "m" is the mole ratio of M to E and varies from 0.01 to 1.5, M' is $NH_4^+$, $H^+$, alkali metals, alkaline earth metals, transition metals and rare earth metals and mixtures thereof, "n" is the mole ratio of M' to E and has a value of 0.03 to 2.0, "p" is the weighted average valence of M', E is a trivalent element selected from the group consisting of aluminum, gallium, iron, boron and mixtures thereof, "x" is mole ratio of P to E and varies from 0.5 to 2.0, "y" is the mole ratio of Si to E and varies from 0.01 to 1.0 and "z" is the mole ratio of O to E and has a value determined by the equation:

$$z = (p \bullet n + 2 \bullet m + 3 + 5 \bullet x + 4 \bullet y)/2$$

and is characterized in that it has the x-ray diffraction pattern having at least the d-spacings and intensities set forth in Table A:

Table A

| 2Θ | d(Å) | I/I₀ |
|---|---|---|
| 6.81 - 6.61 | 12.98 - 13.34 | m - vs |
| 7.75 - 7.55 | 11.40 - 11.70 | m - vs |
| 13.36 - 13.14 | 6.62 - 6.73 | w - m |
| 13.51 - 13.32 | 6.55 - 6.64 | w - m |
| 14.98 - 14.68 | 5.91 - 6.03 | w |
| 15.51 - 15.34 | 5.71 - 5.77 | w - m |
| 18.99 - 18.79 | 4.67 - 4.72 | w - m |
| 20.26 - 20.07 | 4.38 - 4.42 | w - m |
| 21.50 - 21.24 | 4.13 - 4.18 | w - s |
| 24.10 - 23.84 | 3.69 - 3.73 | w - m |
| 24.30 - 24.03 | 3.66 - 3.70 | w - m |
| 24.57 - 24.23 | 3.62 - 3.67 | w - m |
| 26.83 - 26.51 | 3.32 - 3.36 | w - m |
| 27.59 - 27.42 | 3.23 - 3.25 | w - m |
| 27.86 - 27.59 | 3.20 - 3.23 | w |
| 28.68 - 28.49 | 3.11 - 3.13 | w |
| 28.87 - 28.59 | 3.09 - 3.12 | w |
| 30.06 - 29.76 | 2.97 - 3.00 | w - m |
| 30.27 - 29.96 | 2.95 - 2.98 | w - m |
| 31.03 - 30.70 | 2.88 - 2.91 | w - m |
| 33.80 - 33.54 | 2.65 - 2.67 | w - m |
| 34.06 - 33.80 | 2.63 - 2.65 | w |
| 35.60 - 35.31 | 2.52 - 2.54 | w - m |
| 36.65 - 36.34 | 2.45 - 2.47 | w |
| 38.44 - 38.10 | 2.34 - 2.36 | w |

In one embodiment of the invention, the MAPSO-64 is thermally stable up to a temperature of at least 400°C, and in another embodiment the MAPSO-64 is thermally stable up to a temperature of at least 500°C.

When MAPSO-64 is calcined in air and ambient water vapor, there can be loss of metal from the framework, such as Al, which can alter the x-ray diffraction pattern from that observed for the as-synthesized MAPSO-64 in Table A, see Studies in Surface Science, (2004) vol. 154, p.1324 - 1331. The air-calcined MAPSO-64 materials, MAPSO-64C, are characterized by the empirical formula:

$$H_a M_m^{2+} E P_x Si y O_z$$

where M is at least one divalent metal cation selected from the group consisting of $Be^{2+}$, $Mg^{2+}$, $Zn^{2+}$, $Co^{2+}$, $Mn^{2+}$, $Fe^{2+}$, $Ni^{2+}$, "m" is the mole ratio of M to E and varies from 0.01 to 1.5, H is a proton, "a" is the mole ratio of H to E and has a value of 0.1 to 2.0, E is a trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (a + 2 \bullet m + 3 + 5 \bullet x + 4 \bullet y)/2$$

The crystalline MAPSO-64 material of this invention can be used for separating mixtures of molecular species, removing contaminants through ion exchange and catalyzing various hydrocarbon conversion processes. Separation of molecular species can be based either on the molecular size (kinetic diameter) or on the degree of polarity of the molecular species.

The MAPSO-64 compositions of this invention can also be used as a catalyst or catalyst support in various hydrocarbon conversion processes. Hydrocarbon conversion processes are well known in the art and include cracking, hydrocracking, alkylation of both aromatics and isoparaffin, isomerization, polymerization, reforming, hydrogenation, dehydrogenation, transalkylation, dealkylation, hydration, dehydration, hydrotreating, hydrodenitrogenation, hydrodesulfurization, methanol to olefins, methanation and syngas shift process. Specific reaction conditions and the types of feeds which can be used in these processes are set forth in US 4,310,440, US 4,440,871 and US 5,126,308, which are hereby incorporated by reference. Preferred hydrocarbon conversion processes are those in which hydrogen is a component such as hydrotreating or hydrofining, hydrogenation, hydrocracking, hydrodenitrogenation, hydrodesulfurization, etc.

Hydrocracking conditions typically include a temperature in the range of 400° to 1200°F (204-649°C), preferably between 600° and 950°F (316-510°C). Reaction pressures are in the range of atmospheric to 3,500 psig (24,132 kPa g), preferably between 200 and 3000 psig (1379 - 20,685 kPa g). Contact times usually correspond to liquid hourly space velocities (LHSV) in the range of 0.1 hr$^{-1}$ to 15 hr$^{-1}$, preferably between 0.2 and 3 hr$^{-1}$. Hydrogen circulation rates are in the range of 1,000 to 50,000 standard cubic feet (scf) per barrel of charge (178-8,888 std. m$^3$/m$^3$), preferably between 2,000 and 30,000 scf per barrel of charge (355-5,333 std. m$^3$/m$^3$). Suitable hydrotreating conditions are generally within the broad ranges of hydrocracking conditions set out above.

The reaction zone effluent is normally removed from the catalyst bed, subjected to partial condensation and vapor-liquid separation and then fractionated to recover the various components thereof. The hydrogen, and if desired some or all of the unconverted heavier materials, are recycled to the reactor. Alternatively, a two-stage flow may be employed with the unconverted material being passed into a second reactor. Catalysts of the subject invention may be used in just one stage of such a process or may be used in both reactor stages.

Catalytic cracking processes are preferably carried out with the MAPSO-64 composition using feedstocks such as gas oils, heavy naphthas, deasphalted crude oil residua, etc. with gasoline being the principal desired product. Temperature conditions of 850° to 1100°F, LHSV values of 0.5 to 10 and pressure conditions of from 0 to 50 psig are suitable.

Alkylation of aromatics usually involves reacting an aromatic ($C_2$ to $C_{12}$), especially benzene, with a monoolefin to produce a linear alkyl substituted aromatic. The process is carried out at an aromatic: olefin (e.g., benzene: olefin) ratio of between 5:1 and 30:1, a LHSV of 0.3 to 6 hr$^{-1}$, a temperature of 100° to 250°C and pressures of 200 to 1000 psig. Further details on apparatus may be found in US 4,870,222 which is incorporated by reference.

Alkylation of isoparaffins with olefins to produce alkylates suitable as motor fuel components is carried out at temperatures of -30° to 40°C, pressures from atmospheric to 6,894 kPa (1,000 psig) and a weight hourly space velocity (WHSV) of 0.1 to 120. Details on paraffin alkylation may be found in US 5,157,196 and US 5,157,197, which are incorporated by reference.

The conversion of methanol to olefins is effected by contacting the methanol with the MAPSO-64 catalyst at conversion conditions, thereby forming the desired olefins. The methanol can be in the liquid or vapor phase with the vapor phase being preferred. Contacting the methanol with the MAPSO-64 catalyst can be done in a continuous mode or a batch mode with a continuous mode being preferred. The amount of time that the methanol is in contact with the MAPSO-64 catalyst must be sufficient to convert the methanol to the desired light olefin products. When the process is carried out in a batch process, the contact time varies from 0.001 hrs to 1 hr and preferably from 0.01 hr to 1. 0 hr. The longer contact times are used at lower temperatures while shorter times are used at higher temperatures. Further, when the process is carried out in a continuous mode, the Weight Hourly Space Velocity (WHSV) based on methanol can vary from 1 hr$^{-1}$ to 1000 hr$^{-1}$ and preferably from 1hr$^{-1}$ to 100 hr$^{-1}$.

Generally, the process must be carried out at elevated temperatures in order to form light olefins at a fast enough rate. Thus, the process should be carried out at a temperature of 300 °C to 600 °C, preferably from 400 °C to 550 °C and most preferably from 450 °C to 525 °C. The process may be carried out over a wide range of pressure including autogenous pressure. Thus, the pressure can vary from 0 kPa (0 psig) to 1724 kPa (250 psig) and preferably from 34 kPa (5 psig) to 345 kPa (50 psig).

Optionally, the methanol feedstock may be diluted with an inert diluent in order to more efficiently convert the methanol to olefins. Examples of the diluents which may be used are helium, argon, nitrogen, carbon monoxide, carbon dioxide, hydrogen, steam, paraffinic hydrocarbons, e. g., methane, aromatic hydrocarbons, e. g., benzene, toluene and mixtures thereof. The amount of diluent used can vary considerably and is usually from 5 to 90 mole percent of the feedstock and preferably from 25 to 75 mole percent.

The actual configuration of the reaction zone may be any known catalyst reaction apparatus known in the art. Thus, a single reaction zone or a number of zones arranged in series or parallel may be used. In such reaction zones the methanol feedstock is flowed through a bed containing the MAPSO-64 catalyst. When multiple reaction zones are used, one or more MAPSO-64 catalysts may be used in series to produce the desired product mixture. Instead of a fixed bed, a dynamic bed system, e. g., fluidized or moving, may be used. Such a dynamic system would facilitate any regeneration of the MAPSO-64 catalyst that may be required. If regeneration is required, the MAPSO-64 catalyst can be continuously introduced as a moving bed to a regeneration zone where it can be regenerated by means such as oxidation in an oxygen containing atmosphere to remove carbonaceous materials.

The following examples are presented in illustration of this invention and are not intended as undue limitations on the generally broad scope of the invention as set out in the appended claims. The products will be designated as MeAPSO-64 to reflect the composition and structure.

The structure of the MAPSO-64 compositions of this invention was determined by the analysis of x-ray powder patterns. The x-ray patterns presented in the following examples were obtained using standard x-ray powder diffraction techniques. The radiation source was a high-intensity, x-ray tube operated at 45 kV and 35 mA. The diffraction pattern from the copper K-alpha radiation was obtained by appropriate computer based techniques. Flat compressed powder samples were continuously scanned at 2° to 56° (2θ). Interplanar spacings (d) in Angstrom units were obtained from the position of the diffraction peaks expressed as θ where θ is the Bragg angle as observed from digitized data. Intensities were determined from the integrated area of diffraction peaks after subtracting background, "I$_o$" being the intensity of the strongest line or peak, and "I" being the intensity of each of the other peaks.

As will be understood by those skilled in the art the determination of the parameter 2θ is subject to both human and mechanical error, which in combination can impose an uncertainty of $\pm0.4°$ on each reported value of 2θ. This uncertainty is, of course, also manifested in the reported values of the d-spacings, which are calculated from the 2θ values. This imprecision is general throughout the art and is not sufficient to preclude the differentiation of the present crystalline materials from each other and from the compositions of the prior art. In some of the x-ray patterns reported, the relative intensities of the d-spacings are indicated by the notations vs, s, m, and w which represent very strong, strong, medium, and weak, respectively. In terms of 100 x I/I$_o$, the above designations are defined as:

$$w = 0\text{-}15;\ m = 15\text{-}60:\ s = 60\text{-}80\ and\ vs = 80\text{-}100$$

In certain instances the purity of a synthesized product may be assessed with reference to its x-ray powder diffraction pattern. Thus, for example, if a sample is stated to be pure, it is intended only that the x-ray pattern of the sample is free of lines attributable to crystalline impurities, not that there are no amorphous materials present.

In order to more fully illustrate the invention, the following examples are set forth. It is to be understood that the examples are only by way of illustration and are not intended as an undue limitation on the broad scope of the invention as set forth in the appended claims.

EXAMPLE 1

A Teflon beaker was charged 100.00g DEDMAOH (20 %), which was then stirred with a high-speed mixer. Then 4.65 g Al(OH)$_3$ (78.1 %) was added a bit at a time with stirring until the reaction mixture was nearly a clear solution. Next 5.94 g TEOS (98%) was added quickly via dropper. The reaction mixture was stirred for 1.5 hours to give the TEOS a chance to hydrolyze. The reaction mixture was then treated with 12.89 g $H_3PO_4$ (85 %), added dropwise over a 15 minute period and allowed to stir for an additional 20 minutes, at which point the solution was clear. Separately, 20.5 g $Zn(OAc)_2 \cdot 2H_2O$ was dissolved in 10.00g de-ionized water. This solution was added slowly, dropwise, and the reaction mixture remained a clear solution. The reaction mixture was stirred for an additional hour before it was distributed among 7 Teflon-lined autoclaves, and digested at autogenous pressures at various temperatures and time periods. The products were isolated and washed with de-ionized water by centrifugation. The products resulting from reaction mixtures digested at 150°C for 55 and 152 hours and 175°C for 55 hours were identified as ZAPSO-64 by powder x-ray diffraction. Table 1 below shows representative diffraction lines for the 150°C/152 hour phase. Elemental analysis of this product had the elemental ratios C/N = 6.38, N/A1 = 0.40, Zn/Al = 0.97, P/Al = 1.59, Si/Al = 0.16, consistent with the stoichiometry $DEDMA_{0.40}Zn_{0.96}AlP_{1.59}Si_{0.16}O_{6.96}$.

Table 1

| 2-Θ | d(Å) | I/I$_0$(%) |
|---|---|---|
| 6.66 | 13.26 | vs |
| 7.60 | 11.63 | m |
| 13.22 | 6.69 | w |
| 13.36 | 6.62 | m |
| 14.80 | 5.98 | w |
| 15.40 | 5.75 | w |
| 16.66 | 5.32 | w |
| 18.84 | 4.71 | m |
| 20.12 | 4.41 | m |
| 20.26 | 4.38 | w |
| 20.35 | 4.36 | w |
| 21.32 | 4.16 | w |
| 23.93 | 3.72 | w |
| 24.16 | 3.68 | w |
| 24.34 | 3.65 | w |
| 26.64 | 3.34 | w |
| 26.92 | 3.31 | w |
| 27.48 | 3.24 | w |
| 27.72 | 3.22 | w |
| 28.54 | 3.12 | w |
| 28.68 | 3.11 | w |
| 29.88 | 2.99 | w |
| 30.10 | 2.97 | w |
| 30.80 | 2.90 | w |
| 33.64 | 2.66 | w |
| 33.90 | 2.64 | w |
| 35.44 | 2.53 | w |
| 36.48 | 2.46 | w |
| 38.19 | 2.35 | w |
| 43.50 | 2.08 | w |

EXAMPLE 2

A Teflon beaker was charged with 100.00 g ETMAOH (20 %) and placed under a high speed stirrer. With vigorous stirring, 5.27 g Al(OH)$_3$ (78.1 %) slowly added and this dissolved readily. This was followed by the addition of 6.74 g TEOS (98 %) in a single pour. After stirring for 30 minutes to hydrolyze the TEOS, 14.61 g H$_3$PO$_4$ (85 %) was added dropwise. The result was a clear solution. Separately, 2.32 g Zn(OAc)$_2$*2H$_2$O was dissolved in 10.00 g de-ionized water. This solution was added dropwise to the reaction mixture which remained a clear solution. After further homogenization, the reaction mixture was distributed among 7 teflon-lined autoclaves and digested at autogenous pressure at various temperatures and time periods. The products were isolated and washed with de-ionized water by centrifugation. The product resulting from the 125°C/180 hour digestion was identified as MAPSO-64 as determined by powder x-ray diffraction. Table 2 below shows representative diffraction lines for this product.

Table 2

| 2-Θ | d(Å) | I/I$_0$(%) |
|---|---|---|
| 6.76 | 13.06 | m |
| 7.70 | 11.47 | vs |
| 13.30 | 6.65 | m |
| 13.45 | 6.58 | m |
| 14.90 | 5.94 | w |
| 15.40 | 5.75 | w |
| 15.50 | 5.71 | w |
| 18.92 | 4.69 | m |
| 20.21 | 4.39 | m |
| 20.32 | 4.37 | m |
| 21.42 | 4.15 | m |
| 24.05 | 3.70 | w |
| 24.22 | 3.67 | m |
| 24.43 | 3.64 | w |
| 26.72 | 3.33 | m |
| 27.52 | 3.24 | m |
| 27.80 | 3.21 | w |
| 28.62 | 3.12 | w |
| 28.75 | 3.10 | w |
| 29.98 | 2.98 | m |
| 30.19 | 2.96 | m |
| 30.94 | 2.89 | w |
| 31.19 | 2.87 | w |
| 33.72 | 2.66 | m |
| 34.02 | 2.63 | w |
| 35.52 | 2.53 | w |
| 36.54 | 2.46 | w |
| 38.30 | 2.35 | w |
| 43.48 | 2.08 | w |
| 49.28 | 1.85 | w |

(continued)

| 2-Θ | d(Å) | I/I_0(%) |
|---|---|---|
| 49.93 | 1.82 | w |

EXAMPLE 3

A Teflon beaker was charged with 130.00 g ETMAOH (20 %) and 6.19 g colloidal silica (Ludox AS-40, 40 % $SiO_2$) and stirred briefly before it was placed in a Teflon bottle and digested at 95°C for 2 hours. The solution was cooled and 6.29 g $Al(OH)_3$ (85.1 %) was added slowly with vigorous stirring. The reaction mixture was homogenized for 45 minutes post-addition and never clarified, remaining hazy. This was followed by the drop-wise addition of 19.00 g $H_3PO_4$ (85%). The resulting reaction mixture is only semi-transparent. Separately, 1.51 g $Zn(OAc)_2 \cdot 2H_2O$ in 6.0 g de-ionized water. This solution was added to the vigorously stirred reaction mixture in a drop-wise fashion with the homogenous reaction mixture becoming more opaque during the addition. The reaction mixture was divided among 7 Teflon-lined autoclaves and digested at autogenous pressure at various temperatures and time periods. The products were isolated and washed with de-ionized water by centrifugation. The product resulting from the 150°C/47 hour digestion was identified as ZAPSO-64 by powder x-ray diffraction, but the sample contained a slight MAPSO-59 impurity. Table 3 below shows the representative diffraction lines for the MAPSO-64 product.

Table 3

| 2-Θ | d(Å) | I/I_0(%) |
|---|---|---|
| 6.67 | 13.24 | m |
| 7.60 | 11.62 | vs |
| 8.98 | 9.84 | w* |
| 13.20 | 6.70 | m |
| 13.36 | 6.62 | m |
| 14.77 | 5.99 | w |
| 15.38 | 5.76 | m |
| 18.08 | 4.90 | w* |
| 18.84 | 4.71 | m |
| 20.14 | 4.41 | m |
| 20.76 | 4.28 | w* |
| 21.32 | 4.16 | m |
| 22.44 | 3.96 | w* |
| 23.96 | 3.71 | w |
| 24.16 | 3.68 | m |
| 24.34 | 3.65 | w |
| 26.60 | 3.35 | m |
| 27.46 | 3.25 | w |
| 27.70 | 3.22 | w |
| 28.55 | 3.12 | w |
| 28.70 | 3.11 | w |
| 29.90 | 2.99 | m |
| 30.12 | 2.96 | m |
| 30.86 | 2.90 | w |

(continued)

| 2-Θ | d(Å) | I/I$_0$(%) |
|---|---|---|
| 31.16 | 2.87 | w |
| 33.66 | 2.66 | w |
| 33.93 | 2.64 | w |
| 35.44 | 2.53 | w |
| 36.52 | 2.46 | w |
| 38.24 | 2.35 | w |
| 43.40 | 2.08 | w |
| *- MAPSO-59 impurity | | |

EXAMPLE 4

A Teflon bottle was charged with 193.3 g DEDMAOH (20 %) and 8.12 g colloidal silica (Ludox AS-40, 40 % SiO$_2$), sealed and placed in an oven at 100°C for 1.5 hours. The resulting DEDMA silicate solution was placed in a Teflon beaker under a high speed stirrer. Then 8.37 g Al(OH)$_3$ (83.0 %) was added with vigorous stirring. Next 24.95 g H$_3$PO$_4$ (85%) was added drop-wise and the reaction mixture further homogenized. Separately, 1.98 g Zn(OAc)$_2$*2H$_2$O was dissolved in 13.29 g de-ionized water. This solution was added drop-wise to the reaction mixture, which was further homogenized for 30 minutes. The final reaction mixture was a transparent white suspension. A portion of the reaction mixture was placed in a Teflon-lined autoclave and digested at 125°C for 161 hours. The product was isolated and washed with de-ionized water via centrifugation. The product was identified as MAPSO-64 by powder x-ray diffraction. Representative diffraction lines for the product are shown in Table 4 below.

Table 4

| 2-Θ | d(Å) | I/I$_0$(%) |
|---|---|---|
| 6.70 | 13.19 | m |
| 7.62 | 11.60 | vs |
| 10.13 | 8.73 | w |
| 13.20 | 6.70 | m |
| 13.39 | 6.61 | m |
| 14.84 | 5.97 | w |
| 15.44 | 5.74 | m |
| 16.66 | 5.32 | w |
| 18.86 | 4.70 | m |
| 20.18 | 4.40 | m |
| 21.34 | 4.16 | m |
| 23.98 | 3.71 | w |
| 24.18 | 3.68 | m |
| 24.34 | 3.65 | m |
| 26.64 | 3.34 | w |
| 26.98 | 3.30 | w |
| 27.46 | 3.25 | m |
| 27.72 | 3.22 | w |
| 28.54 | 3.12 | w |

(continued)

| 2-Θ | d(Å) | I/I_0(%) |
|---|---|---|
| 28.71 | 3.11 | w |
| 29.90 | 2.99 | m |
| 30.18 | 2.96 | m |
| 30.82 | 2.90 | m |
| 33.66 | 2.66 | w |
| 33.99 | 2.64 | w |
| 35.46 | 2.53 | w |
| 36.42 | 2.47 | w |
| 36.56 | 2.46 | w |
| 38.26 | 2.35 | w |
| 39.07 | 2.30 | w |
| 39.82 | 2.26 | w |
| 40.39 | 2.23 | w |
| 43.42 | 2.08 | w |
| 43.65 | 2.07 | w |
| 49.88 | 1.83 | w |
| 55.69 | 1.65 | w |

EXAMPLE 5

A Teflon beaker was charged with 130.00 g ETMAOH (20 %) and 6.19 g colloidal silica (Ludox AS-40, 40 % $SiO_2$), the mixture was stirred briefly, transferred to a Teflon bottle, and digested at 95°C for 2 hours. The resulting ETMA silicate solution was placed in a beaker under a high speed stirrer and 6.29 g $Al(OH)_3$ (85.1 %) was added slowly, resulting in a cloudy reaction mixture, even after 45 minutes of post addition homogenization. Next, 19.00 g $H_3PO_4$ (85 %) was added intermittently in a drop-wise fashion. A semi-transparent reaction mixture results. Separately, 3.01 g $Zn(OAc)_2$*$2H_2O$ was dissolved in 12.0 g de-ionized water. This solution was added intermittently in three different additions in a drop-wise fashion while the reaction mixture was vigorously stirred. The reaction mixture remained homogenous, but became more opaque. The reaction mixture was loaded into 7 Teflon-lined autoclaves and digested at autogenous pressure at various temperatures and time periods. The products were isolated and washed with de-ionized water by centrifugation. The product resulting from the 125°C/183 hour digestion was identified as MAPSO-64 by powder x-ray diffraction. The representative diffraction lines for this product are shown in Table 5 below.

Table 5

| 2-Θ | d(Å) | I/I_0(%) |
|---|---|---|
| 6.68 | 13.22 | m |
| 7.62 | 11.59 | vs |
| 13.22 | 6.69 | m |
| 13.38 | 6.61 | m |
| 14.83 | 5.97 | w |
| 15.41 | 5.75 | w |
| 18.86 | 4.70 | m |
| 20.14 | 4.41 | m |

(continued)

| 2-Θ | d(Å) | I/I$_0$(%) |
|---|---|---|
| 20.26 | 4.38 | m |
| 21.36 | 4.16 | s |
| 23.98 | 3.71 | w |
| 24.15 | 3.68 | m |
| 24.36 | 3.65 | m |
| 26.68 | 3.34 | m |
| 27.48 | 3.24 | m |
| 27.74 | 3.21 | w |
| 28.55 | 3.12 | w |
| 28.72 | 3.11 | w |
| 29.92 | 2.98 | m |
| 30.14 | 2.96 | m |
| 30.86 | 2.90 | m |
| 33.70 | 2.66 | w |
| 33.97 | 2.64 | w |
| 35.48 | 2.53 | w |
| 36.50 | 2.46 | w |
| 38.26 | 2.35 | w |
| 40.43 | 2.23 | w |
| 43.50 | 2.08 | w |

One embodiment is a microporous crystalline silicometallophosphate material having a three-dimensional framework of $EO_2^-$, $PO_2^+$, $[M^{2+}O_2]^{2-}$ and $SiO_2$ tetrahedral units and an empirical composition in the as synthesized and anhydrous basis expressed by an empirical formula of:

$$R_r M^{2+}_m E P_x Si_y O_z$$

where M is at least one divalent cation selected from the group consisting of alkaline earth and transition metals, "m" is the mole ratio of M to E and varies from 0.01 to 1.5, R is an organoammonium cation selected from the group of ethyltrimethylammonium (ETMA$^+$), diethyldimethylammonium (DEDMA$^+$) and mixtures thereof, "r" is the mole ratio of R to E and has a value of 0.1 to 2, E is an trivalent element selected from the group consisting of aluminum, gallium, iron, boron and mixtures thereof, "x" is the mole ratio of P to E and has a value of 0.5 to 2.5, "y" is the mole ratio of Si to E and varies from 0.01 to 1.0 and "z" is the mole ratio of O to E and has a value determined by the equation:

$$z = (2 \bullet m + r + 3 + 5 \bullet x + 4 \bullet y)/2$$

and is characterized in that it has the x-ray diffraction pattern having at least the d-spacings and intensities set forth in Table A above. E may be aluminum. M may be selected from the group consisting of magnesium, zinc, cobalt, manganese, and mixtures thereof. The silicometallophosphate material may be thermally stable up to a temperature of at least 400°C. The silicometallophosphate material may be thermally stable up to a temperature of at least 500°C. R may be DEDMA$^+$, ETMA$^+$ or mixtures thereof.

The application discloses a process for preparing a microporous crystalline silicometallophosphate material having a three-dimensional framework of at least $EO_2^-$, $PO_2^+$, $[M^{2+}O_2]^{2-}$ and $SiO_2$ tetrahedral units and an empirical composition in the as synthesized and anhydrous basis expressed by an empirical formula of:

$$R_r M_m^{2+} E P_x Si_y O_z$$

where M is at least one divalent cation selected from the group consisting of alkaline earth and transition metals, "m" is the mole ratio of M to E and varies from 0.01 to 1.5, R is an organoammonium cation selected from the group consisting of ethyltrimethylammonium ($ETMA^+$), diethyldimethylammonium ($DEDMA^+$) cations and mixtures thereof, "r" is the mole ratio of R to E and has a value of 0.1 to 2.0, E is a trivalent element selected from the group consisting of aluminum, gallium, iron, boron and mixtures thereof, "x" is the mole ratio of P to E and varies from 0.5 to 2.5, "y" is the mole ratio of Si to E and varies from 0.01 to 1.0 and "z" is the mole ratio of O to E and has a value determined by the equation:

$$z = (2 \bullet m + r + 3 + 5 \bullet x + 4 \bullet y)/2$$

and is characterized in that it has the x-ray diffraction pattern having at least the d-spacings and intensities set forth in Table A above, the process comprising forming a reaction mixture containing reactive sources of R, E, P, M and Si and heating the reaction mixture at a temperature of 60°C to 200°C, for a time sufficient to form the silicometallophosphate material, the reaction mixture having a composition expressed in terms of mole ratios of the oxides of:

$$aR_2O : bMO : E_2O_3 : cP_2O_5 : dSiO_2 : eH_2O$$

where "a" has a value of 0.75 to 16, "b" has a value of 0.01 to 3.0, "c" has a value of 0.8 to 8, "d" has a value of 0.01 to 4, and "e" has a value of 20 to 800. M may be selected from the group consisting of magnesium, zinc, cobalt, manganese, nickel, iron (II) and mixtures thereof. The source of M may be selected from the group consisting of halide salts, nitrate salts, acetate salts, sulfate salts and mixtures thereof. The source of E may be selected from the group consisting of aluminum isopropoxide, aluminum sec-butoxide, precipitated alumina, Al(OH)3, aluminum metal, aluminum salts, boric acid, precipitated gallium oxyhydroxide, gallium sulfate, ferric sulfate, ferric chloride and mixtures thereof. The silicon source maybe selected from the group consisting of tetraethylorthosilicate, fumed silica, colloidal silica and precipitated silica. The reaction mixture may be reacted at a temperature of 125°C to 175°C for a time of 1 day to 7 days. R may be $DEDMA^+$, $ETMA^+$ and mixtures thereof. The process may further comprise adding MAPSO-64 seeds to the reaction mixture.

An embodiment involves a modified form of the silicometallophosphate, where the modification may comprise ammonia calcination under ammonia calcination conditions, and optionally an additional ion-exchange to form a modified microporous silicometallophosphate, MAPSO-64M, having a three-dimensional framework of $EO_2^-$, $PO_2^+$, $[M^{2+}O_2]^{2-}$ and $SiO_2$ tetrahedral units the composition given on an anhydrous basis by the empirical formula:

$$M'^{p+}_n M_m^{2+} E P_x Si_y O_z$$

where M is at least one divalent metal cation selected from the group consisting of $Be^{2+}$, $Mg^{2+}$, $Zn^{2+}$, $Co^{2+}$, $Mn^{2+}$, $Fe^{2+}$, $Ni^{2+}$, and mixtures thereof, "m" is the mole ratio of M to E and varies from 0.01 to 1.5, M' is $NH_4^+$, $H^+$, alkali metals, alkaline earth metals, and rare earth metals and mixtures thereof, "n" is the mole ratio of M' to E and has a value of 0.03 to 2.0, "p" is the weighted average valence of M', E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (p \bullet n + 2 \bullet m + 3 + 5 \bullet x + 4 \bullet y)/2$$

and is characterized in that it has the x-ray diffraction pattern having at least the d-spacings and intensities set forth in Table A above.

An embodiment involves a stable, calcined form of the crystalline microporous silicometallophosphate, MAPSO-64C, the calcination comprising heating the silicometallophosphate under calcination conditions to remove the organic cations in the presence of air and ambient water vapor, said MAPSO-64 having a three-dimensional framework of $EO_2^-$, $PO_2^+$, $SiO_2$ and $[M^{2+}O_2]^{2-}$ tetrahedral units characterized by the empirical formula:

$$H_a M_m^{2+} E P_x Si_y O_z$$

where M is at least one divalent metal cation selected from the group consisting of $Be^{2+}$, $Mg^{2+}$, $Zn^{2+}$, $Co^{2+}$, $Mn^{2+}$, $Fe^{2+}$,

$Ni^{2+}$, and mixtures thereof, "m" is the mole ratio of M to E and varies from 0.01 to 1.0, H is a proton, "a" is the mole ratio of H to E and has a value of 0.1 to 2.0, E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (a + 2 \bullet m + 3 + 5 \bullet x + 4 \bullet y)/2.$$

One embodiment involves hydrocarbon conversion process comprising contacting a stream comprising at least one hydrocarbon with a catalyst at hydrocarbon conversion conditions to generate at least one converted product, wherein the catalyst is selected from the group consisting of a microporous crystalline MAPSO-64 material, a microporous crystalline MAPSO-64M material, a microporous crystalline MAPSO-64C material, or mixtures thereof, where MAPSO-64 has a three-dimensional framework of $EO_2^-$, $PO_2^+$, $[M^{2+}O_2]^{2-}$ and $SiO_2$ tetrahedral units and an empirical composition in the as synthesized and anhydrous basis expressed by an empirical formula of:

$$R_r M_m^{2+} E P_x Si_y O_z$$

where M is at least one cation of valence 2+ selected from the group consisting of alkaline earth and transition metals, "m" is the mole ratio of M to E and varies from 0.01 to 1.5, R is an organoammonium cation selected from the group of ethyltrimethylammonium ($ETMA^+$), diethyldimethylammonium ($DEDMA^+$) and mixtures thereof, "r" is the mole ratio of R to E and has a value of 0.1 to 2.0, E is a trivalent element selected from the group consisting of aluminum, gallium, iron, boron and mixtures thereof, "x" is the mole ratio of P to E and varies from 0.5 to 2.5, "y" is the mole ratio of Si to E and varies from 0.01 to 1.0 and "z" is the mole ratio of O to E and has a value determined by the equation:

$$z = (2 \bullet m + r + 3 + 5 \bullet x + 4 \bullet y)/2$$

and is characterized in that it has the x-ray diffraction pattern having at least the d-spacings and intensities set forth in Table A above; where the microporous crystalline MAPSO-64M material comprises a three-dimensional framework of $EO_2^-$, $PO_2^+$, $[M^{2+}O_2]^{2-}$ and $SiO_2$ tetrahedral units the composition given by the empirical formula:

$$M'_n{}^{p+} M_m^{2+} E P_x Si_y O_z$$

where M is at least one metal cation of valence +2 selected from the group consisting of $Be^{2+}$, $Mg^{2+}$, $Zn^{2+}$, $Co^{2+}$, $Mn^{2+}$, $Fe^{2+}$, $Ni^{2+}$, "m" is the mole ratio of M to E and varies from 0 to 1.5, M' is selected from the group consisting of $NH_4^+$, $H^+$, alkali metals, alkaline earth metals, rare earth metals, and mixtures thereof, "n" is the mole ratio of M' to E and has a value of 0.03 to 2.0, "p" is the weighted average valence of M', E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (p \bullet n + 2 \bullet m + 3 + 5 \bullet x + 4 \bullet y)/2$$

and is characterized in that it has the x-ray diffraction pattern having at least the d-spacings and intensities set forth in Table A above, and where the microporous crystalline MAPSO-64C material comprises a three-dimensional framework of $EO_2^-$, $PO_2^+$, $SiO_2$ and $[M^{2+}O_2]^{2-}$ tetrahedral units characterized by the empirical formula:

$$H_a M_m^{2+} E P_x Si_y O_z$$

where M is at least one metal cation of valence +2 selected from the group consisting of $Be^{2+}$, $Mg^{2+}$, $Zn^{2+}$, $Co^{2+}$, $Mn^{2+}$, $Fe^{2+}$, $Ni^{2+}$, "m" is the mole ratio of M to E and varies from 0.01 to 1.5, H is a proton, "a" is the mole ratio of H to E and has a value of 0.1 to 2.0, E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (a + 2 \bullet m + 3 + 5 \bullet x + 4 \bullet y)/2.$$

The hydrocarbon conversion process may be selected from the group consisting of cracking, hydrocracking, alkylation, isomerization, polymerization, reforming, hydrogenation, dehydrogenation, transalkylation, dealkylation, hydration, dehydration, hydrotreating, hydrofining, hydrodenitrogenation, hydrodesulfurization, methanol to olefins, methanation, syngas shift process, olefin dimerization, oligomerization, dewaxing, and combinations thereof. The hydrocarbon conversion process maybe hydrocracking or hydrotreating wherein the hydrocracking or hydrotreating may be operated at a temperature in the range of 400° to 1200°F (204-649°C) and a pressure in the range of atmospheric to 3,500 psig (24,132 kPa g). The process may be hydrocracking or hydrotreating operated at a liquid hourly space velocities (LHSV) in the range of 0.1 hr$^{-1}$ to 15 hr$^{-1}$. The process may be hydrocracking or hydrotreating operated at hydrogen circulation rates in the range of 1,000 to 50,000 standard cubic feet (scf) per barrel of charge (178-8,888 std. m$^3$/m$^3$). The process may further comprise removing an effluent comprising the at least one converted product, fractionating the effluent, and recovering at least one converted product. The process may further comprise, subjecting the effluent to partial condensation and vapor-liquid separation prior to fractionation. The process may further comprise recycling at least a portion of the effluent to the catalyst. The hydrocarbon conversion process may comprise two stage operation and the catalyst is present in at least one of the two stages. The hydrocarbon conversion process may be catalytic cracking operated at a temperature in the range of 850° to 1100°F , LHSV values of 0.5 to 10 and a pressure in the range of from 0 to 50 psig. The hydrocarbon stream may be selected from the group consisting of gas oils, heavy naphthas, and deasphalted crude oil residua. The hydrocarbon conversion process may be alkylation of aromatics and the converted product may be at least one linear alkyl substituted aromatic, wherein the process is operated at an aromatic: olefin mole ratio of between 5:1 and 30:1, a LHSV of 0.3 to 6 hr-1, a temperature of 100° to 250°C and a pressures of 200 to 1000 psig. The hydrocarbon conversion process may be alkylation of isoparaffins with olefins and the converted product may be at least one alkylate suitable as a motor fuel component, and wherein the process is operated at a temperature of from -30° to 40°C, a pressure from atmospheric to 6,894 kPa (1,000 psig) and a weight hourly space velocity (WHSV) of 0.1 to 120. The hydrocarbon conversion process may be the conversion of methanol to olefins wherein the process is operated at a temperature of 300 °C to 600 °C and a pressure from 0 kPa (0 psig) to 1724 kPa (250 psig). The methanol may be in the liquid or vapor phase and the operation in a continuous or batch mode. The methanol may be diluted with a diluent selected from the group consisting of helium, argon, nitrogen, carbon monoxide, carbon dioxide, hydrogen, steam, at least one paraffinic hydrocarbon, at least one aromatic hydrocarbon, and mixtures thereof. The catalyst may be located in one or more catalyst zones arranged in series or parallel configuration, and wherein the catalyst may be in fixed beds or fluidized beds. The process may further comprise regenerating the catalyst.

An embodiment of the invention is a separation process comprising contacting at least two components with a material to generate at least one separated component, wherein the material is selected from the group consisting of a microporous crystalline MAPSO-64 material, a microporous crystalline MAPSO-64M material, a microporous crystalline MAPSO-64C material, or mixtures thereof, where MAPSO-64 has a three-dimensional framework of $EO_2^-$, $PO_2^+$, $[M2^+O_2]^{2-}$ and $SiO_2$ tetrahedral units and an empirical composition in the as synthesized and anhydrous basis expressed by an empirical formula of:

$$R_r M_m^{2+} E P_x Si_y O_z$$

where M is at least one cation of valence 2+ selected from the group consisting of alkaline earth and transition metals, "m" is the mole ratio of M to E and varies from 0.01 to 1.5, R is an organoammonium cation selected from the group of ethyltrimethylammonium (ETMA$^+$), diethyldimethylammonium (DEDMA$^+$) and mixtures thereof, "r" is the mole ratio of R to E and has a value of 0.1 to 2.0, E is a trivalent element selected from the group consisting of aluminum, gallium, iron, boron and mixtures thereof, "x" is the mole ratio of P to E and varies from 0.5 to 2.5, "y" is the mole ratio of Si to E and varies from 0.01 to 1.0 and "z" is the mole ratio of O to E and has a value determined by the equation:

$$z = (2 \bullet m + r + 3 + 5 \bullet x + 4 \bullet y)/2$$

and is characterized in that it has the x-ray diffraction pattern having at least the d-spacings and intensities set forth in Table A above; the microporous crystalline MAPSO-64M material comprises a three-dimensional framework of $EO_2^-$, $PO_2^+$ and at least one of $[M^{2+}O_2]^{2-}$ and $SiO_2$ tetrahedral units the composition given by the empirical formula:

$$M'_n^{p+} M_m^{2+} E P_x Si_y O_z$$

where M is at least one metal cation of valence +2 selected from the group consisting of $Be^{2+}$, $Mg^{2+}$, $Zn^{2+}$, $Co^{2+}$, $Mn^{2+}$, $Fe^{2+}$, $Ni^{2+}$, "m" is the mole ratio of M to E and varies from 0.01 to 1.5, M' is selected from the group consisting of $NH_4^+$, $H^+$, alkali metals, alkaline earth metals, rare earth metals and mixtures thereof, "n" is the mole ratio of M' to E and has a value of 0.03 to 2.0, "p" is the weighted average valence of M', E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (p \bullet n + 2 \bullet m + 3 + 5 \bullet x + 4 \bullet y)/2$$

and is characterized in that it has the x-ray diffraction pattern having at least the d-spacings and intensities set forth in Table A above; and the microporous crystalline MAPSO-64C material comprises a three-dimensional framework of $EO_2^-$, $PO_2^+$, $[M^{2+}O_2]^{2-}$ and $SiO_2$ tetrahedral units characterized by the empirical formula:

$$H_a M_m^{2+} E P_x Si_y O_z$$

where M is at least one metal cation of valence +2 selected from the group consisting of $Be^{2+}$, $Mg^{2+}$, $Zn^{2+}$, $Co^{2+}$, $Mn^{2+}$, $Fe^{2+}$, $Ni^{2+}$, "m" is the mole ratio of M to E and varies from 0.01 to 1.5, H is a proton, "a" is the mole ratio of H to E and has a value of 0.1 to 2.0, E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (a + 2 \bullet m + 3 + 5 \bullet x + 4 \bullet y)/2.$$

The separation may be based on molecular size of the components, degree of polarity of the components, or ion exchange of the components with the material.

**Claims**

1. A microporous crystalline silicometallophosphate material designated MAPSO-64 having a three-dimensional framework of $EO_2^-$, $PO_2^+$, $[M^{2+}O_2]^{2-}$ and $SiO_2$ tetrahedral units and an empirical composition in the as synthesized and anhydrous basis expressed by an empirical formula of:

$$R_r M^{2+}_m E P_x Si_y O_z$$

where M is at least one divalent cation selected from the group consisting of alkaline earth and transition metals, "m" is the mole ratio of M to E and varies from 0.01 to 1.5, R is an organoammonium cation selected from the group of ethyltrimethylammonium ($ETMA^+$), diethyldimethylammonium ($DEDMA^+$) and mixtures thereof, "r" is the mole ratio of R to E and has a value of 0.1 to 2, E is an trivalent element selected from the group consisting of aluminum, gallium, iron, boron and mixtures thereof, "x" is the mole ratio of P to E and has a value of 0.5 to 2.5, "y" is the mole ratio of Si to E and varies from 0.01 to 1.0 and "z" is the mole ratio of O to E and has a value determined by the equation:

$$z = (2 \bullet m + r + 3 + 5 \bullet x + 4 \bullet y)/2$$

and is **characterized in that** it has the x-ray diffraction pattern having at least the d-spacings and intensities set forth in Table A:

Table A

| $2\Theta$ | d(Å) | $I/I_0$ |
|---|---|---|
| 6.810 - 6.61 | 12.98 - 13.34 | m - vs |
| 7.75 - 7.55 | 11.40 - 11.70 | m - vs |

(continued)

| 2Θ | d(Å) | I/I$_0$ |
|---|---|---|
| 13.36 - 13.14 | 6.62 - 6.73 | w - m |
| 13.51 - 13.32 | 6.55 - 6.64 | w - m |
| 14.98 - 14.68 | 5.91 - 6.03 | w |
| 15.51 - 15.34 | 5.71 - 5.77 | w - m |
| 18.99 - 18.79 | 4.67 - 4.72 | w - m |
| 20.26 - 20.07 | 4.38 - 4.42 | w - m |
| 21.50 - 21.24 | 4.13 - 4.18 | w - s |
| 24.10 - 23.84 | 3.69 - 3.73 | w - m |
| 24.30 - 24.03 | 3.66 - 3.70 | w - m |
| 24.57 - 24.23 | 3.62 - 3.67 | w - m |
| 26.83 - 26.51 | 3.32 - 3.36 | w - m |
| 27.59 - 27.42 | 3.23 - 3.25 | w - m |
| 27.86 - 27.59 | 3.20 - 3.23 | w |
| 28.68 - 28.49 | 3.11 - 3.13 | w |
| 28.87 - 28.59 | 3.09 - 3.12 | w |
| 30.06 - 29.76 | 2.97 - 3.00 | w - m |
| 30.27 - 29.96 | 2.95 - 2.98 | w - m |
| 31.03 - 30.70 | 2.88 - 2.91 | w - m |
| 33.80 - 33.54 | 2.65 - 2.67 | w - m |
| 34.06 - 33.80 | 2.63 - 2.65 | w |
| 35.60 - 35.31 | 2.52 - 2.54 | w - m |
| 36.65 - 36.34 | 2.45 - 2.47 | w |
| 38.44 - 38.10 | 2.34 - 2.36 | w |

2. The microporous crystalline silicometallophosphate material of claim 1 in which E is aluminum and where M is selected from the group consisting of magnesium, zinc, cobalt, manganese, and mixtures thereof.

3. The microporous crystalline silicometallophosphate material of claim 1 where R is DEDMA$^+$ and ETMA$^+$.

4. The microporous crystalline silicometallophosphate material of claim 1 after modification comprising ammonia calcination under ammonia calcination conditions, and optionally an additional ion-exchange to form a modified microporous silicometallophosphate, MAPSO-64M, having a three-dimensional framework of $EO_2^-$, $PO_2^+$, $[M^{2+}O_2]^{2-}$ and $SiO_2$ tetrahedral units the composition given on an anhydrous basis by the empirical formula:

$$M'^{p+}_n M^{2+}_m EP_x Si_y O_z$$

where M is at least one divalent metal cation selected from the group consisting of $Be^{2+}$, $Mg^{2+}$, $Zn^{2+}$, $Co^{2+}$, $Mn^{2+}$, $Fe^{2+}$, $Ni^{2+}$, and mixtures thereof, "m" is the mole ratio of M to E and varies from 0.01 to 1.5, M' is $NH_4^+$, $H^+$, alkali metals, alkaline earth metals, and rare earth metals and mixtures thereof, "n" is the mole ratio of M' to E and has a value of 0.03 to 2.0, "p" is the weighted average valence of M', E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (p \bullet n + 2 \bullet m + 3 + 5 \bullet x + 4 \bullet y)/2$$

and is **characterized in that** it has the x-ray diffraction pattern having at least the d-spacings and intensities set forth in Table A:

Table A

| $2\Theta$ | d(Å) | $I/I_0$ |
|---|---|---|
| 6.81 - 6.61 | 12.98 - 13.34 | m - vs |
| 7.75 - 7.55 | 11.40 - 11.70 | m - vs |
| 13.36 - 13.14 | 6.62 - 6.73 | w - m |
| 13.51 - 13.32 | 6.55 - 6.64 | w - m |
| 14.98 - 14.68 | 5.91 - 6.03 | w |
| 15.51 - 15.34 | 5.71 - 5.77 | w - m |
| 18.99 - 18.79 | 4.67 - 4.72 | w - m |
| 20.26 - 20.07 | 4.38 - 4.42 | w - m |
| 21.50 - 21.24 | 4.13 - 4.18 | w - s |
| 24.10 - 23.84 | 3.69 - 3.73 | w - m |
| 24.30 - 24.03 | 3.66 - 3.70 | w - m |
| 24.57 - 24.23 | 3.62 - 3.67 | w - m |
| 26.83 - 26.51 | 3.32 - 3.36 | w - m |
| 27.59 - 27.42 | 3.23 - 3.25 | w - m |
| 27.86 - 27.59 | 3.20 - 3.23 | w |
| 28.68 - 28.49 | 3.11 - 3.13 | w |
| 28.87 - 28.59 | 3.09 - 3.12 | w |
| 30.06 - 29.76 | 2.97 - 3.00 | w - m |
| 30.27 - 29.96 | 2.95 - 2.98 | w - m |
| 31.03 - 30.70 | 2.88 - 2.91 | w - m |
| 33.80 - 33.54 | 2.65 - 2.67 | w - m |
| 34.06 - 33.80 | 2.63 - 2.65 | w |
| 35.60 - 35.31 | 2.52 - 2.54 | w - m |
| 36.65 - 36.34 | 2.45 - 2.47 | w |
| 38.44 - 38.10 | 2.34 - 2.36 | w |

5. The microporous crystalline silicometallophosphate material of claim 1 after calcination comprising heating the silicometallophosphate of claim 1 under calcination conditions to remove the organic cations in the presence of air and ambient water vapor to form MAPSO-64C, said MAPSO-64 having a three-dimensional framework of $EO_2^-$, $PO_2^+$, $SiO_2$ and $[M^{2+}O_2]^{2-}$ tetrahedral units **characterized by** the empirical formula:

$$H_a M_m^{2+} EP x Si_y O_z$$

where M is at least one divalent metal cation selected from the group consisting of $Be^{2+}$, $Mg^{2+}$, $Zn^{2+}$, $Co^{2+}$, $Mn^{2+}$, $Fe^{2+}$, $Ni^{2+}$, and mixtures thereof, "m" is the mole ratio of M to E and varies from 0.01 to 1.0, H is a proton, "a" is the mole ratio of H to E and has a value of 0.1 to 2.0, E is an trivalent element selected from the group consisting of aluminum, gallium, iron, boron and mixtures thereof, "x" is mole ratio of P to E and varies from 0.5 to 2.5, "y" is the mole ratio of Si to E and varies from 0.01 to 1.0 and "z" is the mole ratio of O to E and has a value determined by the equation:

$$z = (a + 2 \bullet m + 3 + 5 \bullet x + 4 \bullet y)/2.$$

6. A process of contacting a feed with any of the microporous crystalline silicometallophosphate of claims 1, 4 or 5 wherein the process is a hydrocarbon conversion process or a separation process.

7. The process of claim 6 wherein the hydrocarbon conversion process is selected from the group consisting of cracking, hydrocracking, alkylation, isomerization, polymerization, reforming, hydrogenation, dehydrogenation, transalkylation, dealkylation, hydration, dehydration, hydrotreating, hydrofining, hydrodenitrogenation, hydrodesulfurization, methanol to olefins, methanation, syngas shift process, olefin dimerization, oligomerization, dewaxing, and combinations thereof, or wherein the separation process is based on molecular size of the components, degree of polarity of the components, or ion exchange of the components with the material.

**Patentansprüche**

1. Mikroporöses kristallines Silicometallophosphatmaterial mit der Bezeichnung MAPSO-64 mit einem dreidimensionalen Gerüst aus $EO_2^-$-, $PO_2^+$-, $[M^{2+}O_2]^{2-}$-und $SiO_2$-Tetraedereinheiten und einer durch die folgende Summenformel ausgedrückten empirischen Zusammensetzung in der wie bei der Synthese anfallenden Form und auf wasserfreier Basis:

$$R_r M^{2+}{}_m E P_x Si_y O_z$$

wobei M für mindestens ein zweiwertiges Gerüstkation aus der Gruppe bestehend aus Erdalkali- und Übergangsmetallen steht, "m" für das Molverhältnis von M zu E steht und von 0,01 bis 1,5 variiert, R für ein Organoammoniumkation aus der Gruppe Ethyltrimethylammonium (ETMA$^+$), Diethyldimethylammonium (DEDMA$^+$) und Mischungen davon steht, "r" für das Molverhältnis von R zu E steht und einen Wert von 0,1 bis 2 hat, E für ein dreiwertiges Element aus der Gruppe bestehend aus Aluminium, Gallium, Eisen, Bor und Mischungen davon steht, "x" für das Molverhältnis von P zu E steht und einen Wert von 0,5 bis 2,5 hat, "y" für das Molverhältnis von Si zu E steht und von 0,01 bis 1,0 variiert und "z" für das Molverhältnis von O zu E steht und einen durch die folgende Gleichung bestimmten Wert aufweist:

$$z = (2 \bullet m + r + 3 + 5 \bullet x + 4 \bullet y)/2$$

und **dadurch gekennzeichnet ist, dass** es das Röntgenbeugungsmuster mit mindestens den folgenden d-Abständen und Intensitäten gemäß Tabelle A aufweist:

Tabelle A

| $2\Theta$ | d (Å) | $I/I_0$ |
|---|---|---|
| 6,81-6,61 | 12,98-13,34 | m-vs |
| 7,75-7,55 | 11,40-11,70 | m-vs |
| 13,36-13,14 | 6,62-6,73 | w-m |
| 13,51-13,32 | 6,55-6,64 | w-m |
| 14,98-14,68 | 5,91-6,03 | w |
| 15,51-15,34 | 5,71-5,77 | w-m |
| 18,99-18,79 | 4,67-4,72 | w-m |
| 20,26-20,07 | 4,38-4,42 | w-m |
| 21,50-21,24 | 4,13-4,18 | w-s |

(fortgesetzt)

| 2Θ | d (Å) | I/I$_0$ |
|---|---|---|
| 24,10-23,84 | 3,69-3,73 | w-m |
| 24,30-24,03 | 3,66-3,70 | w-m |
| 24,57-24,23 | 3,62-3,67 | w-m |
| 26,83-26,51 | 3,32-3,36 | w-m |
| 27,59-27,42 | 3,23-3,25 | w-m |
| 27,86-27,59 | 3,20-3,23 | w |
| 28,68-28,49 | 3,11-3,13 | w |
| 28,87-28,59 | 3,09-3,12 | w |
| 30,06-29,76 | 2,97-3,00 | w-m |
| 30,27-29,96 | 2,95-2,98 | w-m |
| 31,03-30,70 | 2,88-2,91 | w-m |
| 33,80-33,54 | 2,65-2,67 | w-m |
| 34,06-33,80 | 2,63-2,65 | w |
| 35,60-35,31 | 2,52-2,54 | w-m |
| 36,65-36,34 | 2,45-2,47 | w |
| 38,44-38,10 | 2,34-2,36 | w |

**2.** Mikroporöses kristallines Silicometallophosphatmaterial nach Anspruch 1, wobei E für Aluminium steht und wobei M aus der Gruppe bestehend aus Magnesium, Zink, Cobalt, Mangan und Mischungen davon ausgewählt ist.

**3.** Mikroporöses kristallines Silicometallophosphatmaterial nach Anspruch 1, wobei R für DEDMA$^+$ und ETMA$^+$ steht.

**4.** Mikroporöses kristallines Silicometallophosphatmaterial nach Anspruch 1 nach Modifizierung umfassend Ammoniak-Calcinierung unter Ammoniak-Calcinierungsbedingungen und gegebenenfalls einen zusätzlichen Ionenaustausch zur Bildung eines modifizierten mikroporösen Silicometallophosphats, MAPSO-64M, mit einem dreidimensionalen Gerüst aus EO$_2^-$-, PO$_2^+$-, [M$^{2+}$O$_2$]$^{2-}$- und SiO$_2$-Tetraedereinheiten der auf wasserfreier Basis durch die folgende Summenformel gegebenen Zusammensetzung:

$$M'^{p+}_n M^{2+}_m EP_x Si_y O_z$$

wobei M für mindestens ein zweiwertiges Gerüstmetallkation aus der Gruppe bestehend aus Be$^{2+}$, Mg$^{2+}$, Zn$^{2+}$, Co$^{2+}$, Mn$^{2+}$, Fe$^{2+}$, Ni$^{2+}$ und Mischungen davon steht, "m" für das Molverhältnis von M zu E steht und von 0,01 bis 1,5 variiert, M' für NH$_4^+$, H$^+$, Alkalimetalle, Erdalkalimetalle und Seltenerdmetalle und Mischungen davon steht, "n" für das Molverhältnis von M' zu E steht und einen Wert von 0,03 bis 2,0 hat, "p" für die gewichtete durchschnittliche Wertigkeit von M' steht, E für ein dreiwertiges Element aus der Gruppe bestehend aus Aluminium, Gallium, Eisen, Bor und Mischungen davon steht, "x" für das Molverhältnis von P zu E steht und von 0,5 bis 2,5 variiert, "y" für das Molverhältnis von Si zu E steht und von 0,01 bis 1,0 variiert und "z" für das Molverhältnis von O zu E steht und einen durch die folgende Gleichung bestimmten Wert aufweist:

$$z = (p \bullet n + 2 \bullet m + 3 + 5 \bullet x + 4 \bullet y)/2$$

und **dadurch gekennzeichnet ist, dass** es das Röntgenbeugungsmuster mit mindestens den folgenden d-Abständen und Intensitäten gemäß Tabelle A aufweist:

Tabelle A

| $2\Theta$ | d (Å) | $I/I_0$ |
|---|---|---|
| 6,81-6,61 | 12,98-13,34 | m-vs |
| 7,75-7,55 | 11,40-11,70 | m-vs |
| 13,36-13,14 | 6,62-6,73 | w-m |
| 13,51-13,32 | 6,55-6,64 | w-m |
| 14,98-14,68 | 5,91-6,03 | w |
| 15,51-15,34 | 5,71-5,77 | w-m |
| 18,99-18,79 | 4,67-4,72 | w-m |
| 20,26-20,07 | 4,38-4,42 | w-m |
| 21,50-21,24 | 4,13-4,18 | w-s |
| 24,10-23,84 | 3,69-3,73 | w-m |
| 24,30-24,03 | 3,66-3,70 | w-m |
| 24,57-24,23 | 3,62-3,67 | w-m |
| 26,83-26,51 | 3,32-3,36 | w-m |
| 27,59-27,42 | 3,23-3,25 | w-m |
| 27,86-27,59 | 3,20-3,23 | w |
| 28,68-28,49 | 3,11-3,13 | w |
| 28,87-28,59 | 3,09-3,12 | w |
| 30,06-29,76 | 2,97-3,00 | w-m |
| 30,27-29,96 | 2,95-2,98 | w-m |
| 31,03-30,70 | 2,88-2,91 | w-m |
| 33,80-33,54 | 2,65-2,67 | w-m |
| 34,06-33,80 | 2,63-2,65 | w |
| 35,60-35,31 | 2,52-2,54 | w-m |
| 36,65-36,34 | 2,45-2,47 | w |
| 38,44-38,10 | 2,34-2,36 | w |

5. Mikroporöses kristallines Silicometallophosphatmaterial nach Anspruch 1 nach Calcinierung umfassend das Erhitzen des Silicometallophosphats nach Anspruch 1 unter Calcinierungsbedingungen zur Entfernung der organischen Kationen in Anwesenheit von Luft und Umgebungswasserdampf zur Bildung von MAPSO-64C, wobei das MAPSO-64C ein dreidimensionales Gerüst aus $EO_2^-$-, $PO_2^+$-, $SiO_2$- und $[M^{2+}O_2]^{2-}$-Tetraedereinheiten aufweist, **gekennzeichnet durch** die Summenformel:

$$H_aM_m^{2+}EP_xSi_yO_z$$

wobei M für mindestens ein zweiwertiges Gerüstmetallkation aus der Gruppe bestehend aus $Be^{2+}$, $Mg^{2+}$, $Zn^{2+}$, $Co^{2+}$, $Mn^{2+}$, $Fe^{2+}$, $Ni^{2+}$ und Mischungen davon steht, "m" für das Molverhältnis von M zu E steht und von 0,01 bis 1,0 variiert, H für ein Proton steht, "a" für das Molverhältnis von H zu E steht und einen Wert von 0,1 bis 2,0 hat, E für ein dreiwertiges Element aus der Gruppe bestehend aus Aluminium, Gallium, Eisen, Bor und Mischungen davon steht, "x" für das Molverhältnis von P zu E steht und von 0,5 bis 2,5 variiert, "y" für das Molverhältnis von Si zu E steht und von 0,01 bis 1,0 variiert und "z" für das Molverhältnis von O zu E steht und einen durch die folgende Gleichung bestimmten Wert aufweist:

$$z = (a + 2 \bullet m + 3 + 5 \bullet x + 4 \bullet y)/2.$$

23

**6.** Verfahren zum Inberührungbringen eines Einsatzstoffs mit einem der mikroporösen kristallinen Silicometallophosphate der Ansprüche 1, 4 oder 5, wobei es sich bei dem Verfahren um ein Kohlenwasserstoffumwandlungsverfahren oder ein Trennverfahren handelt.

**7.** Verfahren nach Anspruch 6, wobei das Kohlenwasserstoffumwandlungsverfahren aus der Gruppe bestehend aus Cracking, Hydrocracking, Alkylierung, Isomerisierung, Polymerisation, Reformierung, Hydrierung, Dehydrierung, Umalkylierung, Dealkylierung, Hydratisierung, Dehydratisierung, Hydrotreating, Hydrofining, Hydrodenitrogenierung, Hydrodesulfurierung, Methanol-To-Olefins, Methanierung, Syngas-Shift-Verfahren, Olefindimerisation, Oligomerisation, Entparaffinierung und Kombinationen davon ausgewählt wird bzw. wobei das Trennverfahren auf der Molekülgröße der Komponenten, dem Polaritätsgrad der Komponenten oder dem Ionenaustausch der Komponenten mit dem Material basiert.

**Revendications**

**1.** Matériau silicométallophosphate cristallin microporeux désigné MAPSO-64 ayant une charpente tridimensionnelle de motifs tétraédriques $EO_2^-$, $PO_2^+$, $[M^{2+}O_2]^{2-}$ et $SiO_2$ et une composition empirique sur la base brute de synthèse et anhydre exprimée par une formule empirique :

$$R_f M^{2+}{}_m E P_x Si_y O_z$$

où M est au moins un cation divalent choisi dans le groupe constitué par les métaux alcalino-terreux et de transition, « m » est le rapport molaire entre M et E et varie de 0,01 à 1,5, R est un cation organoammonium choisi dans le groupe constitué par l'éthyltriméthyl-ammonium (ETMA$^+$), le diéthyldiméthylammonium (DEDMA$^+$) et les mélanges de ceux-ci, « r » est le rapport molaire entre R et E et prend une valeur de 0, 1 à 2, E est un élément trivalent choisi dans le groupe constitué par l'aluminium, le gallium, le fer, le bore et les mélanges de ceux-ci, « x » est le rapport molaire entre P et E et prend une valeur de 0,5 à 2,5, « y » est le rapport molaire entre Si et E et varie de 0,01 à 1,0 et « z » est le rapport molaire entre O et E et prend une valeur déterminée par l'équation :

$$z = (2 \cdot m + r + 3 + 5 \cdot x + 4 \cdot y)/2$$

et est **caractérisé en ce qu'**il a le diagramme de diffraction des rayons X ayant au moins les distances interréticulaires et les intensités indiquées dans le tableau A :

Tableau A

| $2\Theta$ | d (Å) | $I/I_0$ |
|---|---|---|
| 6,81 - 6,61 | 12,98 - 13,34 | m - vs |
| 7,75 - 7,55 | 11,40 - 11,70 | m - vs |
| 13,36 - 13,14 | 6,62 - 6,73 | w - m |
| 13,51 - 13,32 | 6,55 - 6,64 | w - m |
| 14,98 - 14,68 | 5,91 - 6,03 | w |
| 15,51 - 15,34 | 5,71 - 5,77 | w - m |
| 18,99 - 18,79 | 4,67 - 4,72 | w - m |
| 20,26 - 20,07 | 4,38 - 4,42 | w - m |
| 21,50 - 21,24 | 4,13 - 4,18 | w - s |
| 24,10 - 23,84 | 3,69 - 3,73 | w - m |
| 24,30 - 24,03 | 3,66 - 3,70 | w - m |
| 24,57 - 24,23 | 3,62 - 3,67 | w - m |
| 26,83 - 26,51 | 3,32 - 3,36 | w - m |

(suite)

| 2Θ | d (Å) | I/I$_0$ |
|---|---|---|
| 27,59 - 27,42 | 3,23 - 3,25 | w - m |
| 27,86 - 27,59 | 3,20 - 3,23 | w |
| 28,68 - 28,49 | 3,11 - 3,13 | w |
| 28,87 - 28,59 | 3,09 - 3,12 | w |
| 30,06 - 29,76 | 2,97 - 3,00 | w - m |
| 30,27 - 29,96 | 2,95 - 2,98 | w - m |
| 31,03 - 30,70 | 2,88 - 2,91 | w - m |
| 33,80 - 33,54 | 2,65 - 2,67 | w - m |
| 34,06 - 33,80 | 2,63 - 2,65 | w |
| 35,60 - 35,31 | 2,52 - 2,54 | w - m |
| 36,65 - 36,34 | 2,45 - 2,47 | w |
| 38,44 - 38,10 | 2,34 - 2,36 | w |

2. Matériau silicométallophosphate cristallin microporeux de la revendication 1 dans lequel E est l'aluminium et où M est choisi dans le groupe constitué par le magnésium, le zinc, le cobalt, le manganèse, et les mélanges de ceux-ci.

3. Matériau silicométallophosphate cristallin microporeux de la revendication 1 où R est le DEDMA$^+$ et l'ETMA$^+$.

4. Matériau silicométallophosphate cristallin microporeux de la revendication 1 après une modification comprenant une calcination à l'ammoniac dans des conditions de calcination à l'ammoniac, et éventuellement un échange d'ions supplémentaire pour former un silicométallophosphate microporeux modifié, MAPSO-64M, ayant une charpente tridimensionnelle de motifs tétraédriques $EO_2^-$, $PO_2^+$, $[M^{2+}O_2]^{2-}$ et $SiO_2$ la composition donnée sur une base anhydre par la formule empirique :

$$M'_n^{p+}M_m^{2+}EP_xSi_yO_z$$

où M est au moins un cation métallique divalent choisi dans le groupe constitué par $Be^{2+}$, $Mg^{2+}$, $Zn^{2+}$, $Co^{2+}$, $Mn^{2+}$, $Fe^{2+}$, $Ni^{2+}$ et les mélanges de ceux-ci, « m » est le rapport molaire entre M et E et varie de 0,01 à 1,5, M' représente $NH_4^+$, $H^+$, les métaux alcalins, les métaux alcalino-terreux, et les terres rares et les mélanges de ceux-ci, « n » est le rapport molaire entre M' et E et prend une valeur de 0,03 à 2,0, « p » est la valence moyenne pondérée de M', E est un élément trivalent choisi dans le groupe constitué par l'aluminium, le gallium, le fer, le bore et les mélanges de ceux-ci, « x » est le rapport molaire entre P et E et prend une valeur de 0,5 à 2,5, « y » est le rapport molaire entre Si et E et prend une valeur de 0,01 à 1,0 et « z » est le rapport molaire entre O et E et prend une valeur déterminée par l'équation :

$$z = (p \cdot n + 2 \cdot m + 3 + 5 \cdot x + 4 \cdot y)/2$$

et est **caractérisé en ce qu'**il a le diagramme de diffraction des rayons X ayant au moins les distances interréticulaires et les intensités indiquées dans le tableau A :

Tableau A

| 2Θ | d (Å) | I/I$_0$ |
|---|---|---|
| 6, 81 - 6,61 | 12,98 - 13,34 | m - vs |
| 7,75 - 7,55 | 11,40 - 11,70 | m - vs |
| 13,36 - 13,14 | 6,62 - 6,73 | w - m |

(suite)

| 2Θ | d (Å) | I/I$_0$ |
|---|---|---|
| 13,51 - 13,32 | 6,55 - 6,64 | w - m |
| 14,98 - 14,68 | 5,91 - 6,03 | w |
| 15,51 - 15,34 | 5,71 - 5,77 | w - m |
| 18,99 - 18,79 | 4,67 - 4,72 | w - m |
| 20,26 - 20,07 | 4,38 - 4,42 | w - m |
| 21,50 - 21,24 | 4,13 - 4,18 | w - S |
| 24,10 - 23,84 | 3,69 - 3,73 | w - m |
| 24,30 - 24,03 | 3,66 - 3,70 | w - m |
| 24,57 - 24,23 | 3,62 - 3,67 | w - m |
| 26,83 - 26,51 | 3,32 - 3,36 | w - m |
| 27,59 - 27,42 | 3,23 - 3,25 | w - m |
| 27,86 - 27,59 | 3,20 - 3,23 | w |
| 28,68 - 28,49 | 3,11 - 3,13 | w |
| 28,87 - 28,59 | 3,09 - 3,12 | w |
| 30,06 - 29,76 | 2,97 - 3,00 | w - m |
| 30,27 - 29,96 | 2,95 - 2,98 | w - m |
| 31,03 - 30,70 | 2,88 - 2,91 | w - m |
| 33,80 - 33,54 | 2,65 - 2,67 | w - m |
| 34,06 - 33,80 | 2,63 - 2,65 | w |
| 35,60 - 35,31 | 2,52 - 2,54 | w - m |
| 36,65 - 36,34 | 2,45 - 2,47 | w |
| 38,44 - 38,10 | 2,34 - 2,36 | w |

5. Matériau silicométallophosphate cristallin microporeux de la revendication 1 après une calcination comprenant le chauffage du silicométallophosphate de la revendication 1 dans des conditions de calcination pour retirer les cations organiques en présence d'air et de vapeur d'eau ambiante pour former du MAPSO-64C, ledit MAPSO-64 ayant une charpente tridimensionnelle de motifs tétraédriques $EO_2^-$, $PO_2^+$, $SiO_2$ et $[M^{2+}O_2]^{2-}$ **caractérisée par** la formule empirique :

$$H_aM_m^{2+}EP_xSi_yO_z$$

où M est au moins un cation métallique divalent choisi dans le groupe constitué par $Be^{2+}$, $Mg^{2+}$, $Zn^{2+}$, $Co^{2+}$, $Mn^{2+}$, $Fe^{2+}$, $Ni^{2+}$ et les mélanges de ceux-ci, « m » est le rapport molaire entre M et E et varie de 0,01 à 1,0, H est un proton, « a » est le rapport molaire entre H et E et prend une valeur de 0,1 à 2,0, E est un élément trivalent choisi dans le groupe constitué par l'aluminium, le gallium, le fer, le bore et les mélanges de ceux-ci, « x » est le rapport molaire entre P et E et varie de 0,5 à 2,5, « y » est le rapport molaire entre Si et E et varie de 0,01 à 1,0 et « z » est le rapport molaire entre O et E et prend une valeur déterminée par l'équation :

```
z = (a + 2•m + 3 + 5•x + 4•y)/2.
```

6. Procédé de mise en contact d'une charge avec n'importe quel silicométallophosphate cristallin microporeux des revendications 1, 4 ou 5, le procédé étant un procédé de conversion ou un procédé de séparation d'hydrocarbures.

7. Procédé de la revendication 6, le procédé de conversion d'hydrocarbures étant choisi dans le groupe constitué par le craquage, l'hydrocraquage, l'alkylation, l'isomérisation, la polymérisation, le reformage, l'hydrogénation, la déshydrogénation, la transalkylation, la désalkylation, l'hydratation, la déshydratation, l'hydrotraitement, l'hydroraffinage, l'hydrodénitrogénation, l'hydrodésulfuration, la conversion du méthanol en oléfines, la méthanation, le procédé de déplacement pour le gaz de synthèse, la dimérisation d'oléfines, l'oligomérisation, le déparaffinage, et les combinaisons de ceux-ci, ou le procédé de séparation étant basé sur la taille moléculaire des constituants, le degré de polarité des constituants, ou l'échange d'ions des constituants avec le matériau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4310440 A
- US 4440871 A
- US 4567029 A
- US 4973785 A
- EP 0161489 B1
- US 6776975 B1
- US 5126308 A
- US 4870222 A
- US 5157196 A
- US 5157197 A

### Non-patent literature cited in the description

- **PATINEC.** *Chem. Mater.,* 1999, vol. 11, 2456-2462
- *Studies in Surface Science,* 2004, vol. 154, 1324-1331